# EUROPEAN PATENT SPECIFICATION

(11) **EP 0 946 735 B1**
(45) Date of publication and mention of the grant of the patent: **08.02.2006**
(21) Application number: 97948751.9
(22) Date of filing: 18.12.1997
(51) Int. Cl.: C12N 15/81, C12N 15/62, C12N 1/19, C07K 14/62

(54) **N-TERMINALLY EXTENDED PROTEINS EXPRESSED IN YEAST**
N-TERMINAL VERLÄNGERTE PROTEINE EXPRIMIERT IN HEFE
PROTEINES A EXTENSION D'EXTREMITE N-TERMINAL EXPRIMEES DANS LA LEVURE

(30) Priority: 20.12.1996 DK 148296
(43) Date of publication of application: 06.10.1999
(73) Proprietor: NOVO NORDISK A/S, 2880 Bagsvaerd (DK)
(72) Inventor: KJELDSEN, Thomas, B rglum, DK-2830 Virum (DK); HAVELUND, Svend, DK-2880 Bagsv rd (DK); PETTERSSON, Annette, Frost, DK-3520 Farum (DK); BALSCHMIDT, Per, DK-3060 Esperg rde (DK)
(74) Representative: Secher, Anne
(86) International application number: PCT/DK1997/000581
(87) International publication number: WO 1998/028429

(56) References cited:
- WO-A-90/10075
- WO-A-95/34666
- WO-A-95/35384

### SEQUENCE LISTING

<110> Novo Nordisk A/S
   Kjeldsen, Thomas
<120> N-Terminally Extended Proteins Expressed in Yeast
<130> 4956
<160> 53
<170> PatentIn version 3.2
<210> 1
   <211> 45
   <212> PRT
   <213> Artificial
<220>
   <223> artificial
<400> 1
   Gln Pro Ile Asp Asp Thr Glu Ser Asn Thr Thr Ser Val Asn Leu Met
   1               5                   10                  15
   Ala Asp Asp Thr Glu Ser Arg Phe Ala Thr Asn Thr Thr Leu Ala Gly
               20                  25                  30
   Gly Leu Asp Val Val Asn Leu Ile Ser Met Ala Lys Arg
           35                  40                  45
<210> 2
   <211> 43
   <212> PRT
   <213> Artificial
<220>
   <223> artificial
<400> 2
   Gln Pro Ile Asp Asp Thr Glu Ser Asn Thr Thr Ser Val Asn Leu Met
   1               5                   10                  15
   Ala Asp Asp Thr Glu Ser Ala Phe Ala Thr Asn Thr Thr Leu Ala Leu
               20                  25                  30
   Asp Val Val Asn Leu Ile Ser Met Ala Lys Arg
           35                  40
<210> 3
   <211> 43
   <212> PRT
   <213> Artificial
<220>
   <223> artificial
<400> 3
   Ser Pro Ile Asp Asp Thr Glu Ser Asn Thr Thr Ser Val Asn Leu Met
   1               5                   10                  15
   Ala Asp Asp Thr Glu Ser Arg Phe Ala Thr Asn Thr Thr Leu Ala Leu
               20                  25                  30
   Asp Val Val Asn Leu Ile Ser Met Ala Lys Arg
           35                  40
<210> 4
   <211> 45
   <212> PRT
   <213> Artificial
<220>
   <223> artificial
<400> 4
   Gln Pro Ile Asp Asp Thr Glu Ser Asn Thr Thr Ser Val Asn Leu Met
   1               5                   10                  15
   Ala Asp Asp Thr Glu Ser Arg Phe Ala Thr Asn Thr Thr Asn Ser Gly
               20                  25                  30
   Gly Leu Asp Val Val Asn Leu Ile Ser Met Ala Lys Arg
           35                  40                  45
<210> 5
   <211> 45
   <212> PRT
   <213> Artificial
<220>
   <223> artificial
<400> 5
   Gln Pro Ile Asp Asp Thr Glu Ser Asn Thr Thr Ser Val Asn Leu Met
   1 5 10 15
   Ala Asp Asp Thr Glu Ser Arg Phe Ala Thr Asn Thr Thr Ser Val Gly
   20 25 30
   Gly Leu Asp Val Val Asn Leu Ile Ser Met Ala Lys Arg
   35 40 45
<210> 6
   <211> 45
   <212> PRT
   <213> Artificial
<220>
   <223> artificial
<400> 6
   Gln Pro Ile Asp Asp Thr Glu Ser Asn Thr Thr Ser Val Asn Leu Met
   1 5 10 15
   Ala Asp Asp Thr Glu Ser Arg Phe Ala Thr Asn Thr Thr Leu Ala Gly
   20 25 30
   Gly Leu Asp Val Val Gly Leu Ile Ser Met Ala Lys Arg
   35 40 45
<210> 7
   <211> 44
   <212> PRT
   <213> Artificial
<220>
   <223> artificial
<400> 7
   Gln Pro Asp Asp Thr Glu Ser Asn Thr Thr Ser Val Asn Leu Met Ala
   1 5 10 15
   Asp Asp Thr Glu Ser Ala Phe Ala Thr Asn Thr Thr Ser Val Gly Gly
   20 25 30
   Leu Asp Val Val Gly Leu Ile Ser Met Ala Lys Arg
   35 40
<210> 8
   <211> 45
   <212> PRT
   <213> Artificial
<220>
   <223> artificial
<400> 8
   Gln Pro Ile Asp Asp Thr Glu Ser Asn Thr Thr Ser Val Asn Leu Met
   1               5                   10                  15
   Ala Asp Asp Thr Glu Ser Ala Phe Ala Thr Asn Thr Thr Leu Ala Gly
               20                  25                  30
   Gly Leu Asp Val Val Asn Leu Ile Ser Met Ala Lys Arg
               35                  40                  45
<210> 9
   <211> 45
   <212> PRT
   <213> Artificial
<220>
   <223> artificial
<400> 9
   Gln Pro Ile Asp Asp Thr Glu Ser Asn Thr Thr Ser Val Asn Leu Met
   1               5                   10                  15
   Ala Asp Asp Thr Glu Ser Ala Phe Ala Thr Asn Thr Thr Asn Ser Gly
               20                  25                  30
   Gly Leu Asp Val Val Asn Leu Ile Ser Met Ala Lys Arg
           35                  40                  45
<210> 10
   <211> 45
   <212> PRT
   <213> Artificial
<220>
   <223> artificial
<400> 10
   Gln Pro Ile Asp Asp Thr Glu Ser Asn Thr Thr Ser Val Asn Leu Met
   1               5                   10                  15
   Ala Asp Asp Thr Glu Ser Ala Phe Ala Thr Asn Thr Thr Leu Ala Gly
               20                  25                  30
   Gly Leu Asp Val Val Gly Leu Ile Ser Met Ala Lys Arg
           35                  40                  45
<210> 11
   <211> 6
   <212> PRT
   <213> Saccharomyces cerevisiae
<400> 11
   Lys Arg Glu Ala Glu Ala
   1 5
<210> 12
   <211> 6
   <212> PRT
   <213> Artificial
<220>
   <223> artificial
<400> 12
   Glu Glu Gly Glu Pro Lys
   1 5
<210> 13
   <211> 15
   <212> PRT
   <213> Artificial
<220>
   <223> artificial
<400> 13
   Gln Pro Ile Asp Glu Asp Asn Asp Thr Ser Val Asn Leu Pro Ala
   1 5 10 15
<210> 14
   <211> 16
   <212> PRT
   <213> Artificial
<220>
   <223> artificial
<400> 14
   Gln Pro Ile Asp Asn Glu Ser Asn Thr Thr Ser Val Asn Leu Pro Ala
   1 5 10 15
<210> 15
   <211> 16
   <212> PRT
   <213> Artificial
<220>
   <223> artificial
<400> 15
   Gln Pro Ile Asp Asp Glu Ser Asn Thr Thr Ser Val Asn Leu Pro Ala
   1 5 10 15
<210> 16
   <211> 15
   <212> PRT
   <213> Artificial
<220>
   <223> artificial
<400> 16
   Gln Pro Ile Asp Asp Glu Asn Thr Thr Ser Val Asn Leu Pro Val
   1               5                   10                  15
<210> 17
   <211> 16
   <212> PRT
   <213> Artificial
<220>
   <223> artificial
<400> 17
   Gln Pro Ile Asp Asp Thr Glu Asn Thr Thr Ser Val Asn Leu Pro Ala
   1               5                   10                  15
<210> 18
   <211> 17
   <212> PRT
   <213> Artificial
<220>
   <223> artificial
<400> 18
   Gln Pro Ile Asp Asp Thr Glu Ser Asn Thr Thr Ser Val Asn Leu Pro
   1               5                   10                  15
   Ala
<210> 19
   <211> 15
   <212> PRT
   <213> Artificial
<220>
   <223> artificial
<400> 19
   Gln Pro Ile Asp Asp Glu Asn Thr Thr Ser Val Asn Leu Met Ala
   1               5                   10                  15
<210> 20
   <211> 18
   <212> PRT
   <213> Artificial
<220>
   <223> artificial
<400> 20
   Gln Pro Ile Asp Asp Thr Glu Ser Asn Thr Thr Ser Val Asn Leu Pro
   1 5 10 15
   Gly Ala
<210> 21
   <211> 17
   <212> PRT
   <213> Artificial
<220>
   <223> artificial
<400> 21
   Gln Pro Ile Asp Asp Thr Glu Ser Asn Thr Thr Ser Val Asn Leu Met
   1 5 10 15
   Ala
<210> 22
   <211> 17
   <212> PRT
   <213> Artificial
<220>
   <223> artificial
<400> 22
   Gln Pro Ile Asp Asp Thr Glu Ser Asn Thr Thr Ser Val Asn Val Pro
   1 5 10 15
   Thr
<210> 23
   <211> 17
   <212> PRT
   <213> Artificial
<220>
   <223> artificial
<400> 23
   Gln Pro Ile Asp Asp Thr Glu Ser Asn Thr Thr Leu Val Asn Val Pro
   1 5 10 15
   Thr
<210> 24
   <211> 17
   <212> PRT
   <213> Artificial
<220>
   <223> artificial
<400> 24
   Gln Pro Ile Asp Asp Thr Glu Ser Asn Thr Thr Ser Val Asn Leu Pro
   1               5                   10                  15
   Thr
<210> 25
   <211> 18
   <212> PRT
   <213> Artificial
<220>
   <223> artificial
<400> 25
   Gln Pro Ile Asp Asp Thr Glu Ser Asn Thr Thr Leu Val Asn Leu Pro
   1               5                   10                  15
   Gly Ala
<210> 26
   <211> 21
   <212> PRT
   <213> Artificial
<220>
   <223> artificial
<400> 26
   Gln Pro Ile Asp Asp Thr Glu Ser Asn Thr Thr Ser Val Asn Leu Met
   1               5                   10                  15
   Ala Pro Ala Val Ala
               20
<210> 27
   <211> 26
   <212> PRT
   <213> Artificial
<220>
   <223> artificial
<400> 27
   Gln Pro Ile Asp Asp Thr Glu Ser Asn Thr Thr Ser Val Asn Leu Met
   1               5                   10                  15
   Asp Leu Ala Val Val Gly Leu Pro Gly Ala
               20                  25
<210> 28
   <211> 33
   <212> PRT
   <213> Artificial
<220>
   <223> artificial
<400> 28
   Gln Pro Ile Asp Asp Thr Glu Ser Asn Thr Thr Ser Val Asn Leu Met
   1 5 10 15
   Ala Asp Asp Thr Glu Ser Ile Asn Thr Thr Leu Val Asn Leu Pro Gly
   20 25 30
   Ala
<210> 29
   <211> 19
   <212> PRT
   <213> Artificial
<220>
   <223> artificial
<400> 29
   Gln Pro Ile Asp Asp Thr Glu Ser Ile Asn Thr Thr Leu Val Asn Leu
   1 5 10 15
   Pro Gly Ala
<210> 30
   <211> 18
   <212> PRT
   <213> Artificial
<220>
   <223> artificial
<400> 30
   Gln Pro Ile Asp Asp Thr Glu Ser Asn Thr Thr Leu Val Asn Leu Pro
   1 5 10 15
   Gly Ala
<210> 31
   <211> 35
   <212> PRT
   <213> Artificial
<220>
   <223> artificial
<400> 31
   Gln Pro Ile Asp Asp Thr Glu Ser Asn Thr Thr Ser Val Asn Leu Met
   1 5 10 15
   Ala Asp Asp Thr Glu Ser Arg Phe Ala Thr Asn Thr Thr Leu Val Asn
   20 25 30
   Leu Pro Leu
   35
<210> 32
   <211> 36
   <212> PRT
   <213> Artificial
<220>
   <223> artificial
<400> 32
   Gln Pro Ile Asp Asp Thr Glu Ser Asn Thr Thr Ser Val Asn Leu Met
   1 5 10 15
   Ala Asp Asp Thr Glu Ser Ile Asn Thr Thr Leu Val Asn Leu Ala Asn
   20 25 30
   Val Ala Met Ala
   35
<210> 33
   <211> 20
   <212> PRT
   <213> Artificial
<220>
   <223> artificial
<400> 33
   Gln Pro Ile Asp Asp Thr Glu Ser Ala Ile Asn Thr Thr Leu Val Asn
   1 5 10 15
   Leu Pro Gly Ala
   20
<210> 34
   <211> 21
   <212> PRT
   <213> Artificial
<220>
   <223> artificial
<400> 34
   Gln Pro Ile Asp Asp Thr Glu Ser Phe Ala Thr Asn Thr Thr Leu Val
   1 5 10 15
   Asn Leu Pro Gly Ala
   20
<210> 35
   <211> 36
   <212> PRT
   <213> Artificial
<220>
   <223> artificial
<400> 35
   Gln Pro Ile Asp Asp Thr Glu Ser Ile Asn Thr Thr Leu Val Asn Leu
   1 5 10 15
   Met Ala Asp Asp Thr Glu Ser Arg Phe Ala Thr Asn Thr Thr Leu Val
   20 25 30
   Asn Leu Pro Leu
   35
<210> 36
   <211> 39
   <212> PRT
   <213> Artificial
<220>
   <223> artificial
<400> 36
   Gln Pro Ile Asp Asp Thr Glu Ser Ile Asn Thr Thr Leu Val Asn Leu
   1 5 10 15
   Met Ala Asp Asp Thr Glu Ser Arg Phe Ala Thr Asn Thr Thr Leu Asp
   20 25 30
   Val Val Asn Leu Pro Gly Ala
   35
<210> 37
   <211> 21
   <212> PRT
   <213> Artificial
<220>
   <223> artificial
<400> 37
   Gln Pro Ile Asp Asp Thr Glu Ser Ala Ala Ile Asn Thr Thr Leu Val
   1 5 10 15
   Asn Leu Pro Gly Ala
   20
<210> 38
   <211> 39
   <212> PRT
   <213> Artificial
<220>
   <223> artificial
<400> 38
   Gln Pro Ile Asp Asp Thr Glu Ser Asn Thr Thr Ser Val Asn Leu Met
   1 5 10 15
   Ala Asp Asp Thr Glu Ser Arg Phe Ala Thr Asn Thr Thr Leu Val Asn
   20 25 30
   Leu Ala Asn Val Ala Met Ala
   35
<210> 39
   <211> 39
   <212> PRT
   <213> Artificial
<220>
   <223> artificial
<400> 39
   Gln Pro Ile Asp Asp Thr Glu Ser Asn Thr Thr Ser Val Asn Leu Met
   1 5 10 15
   Ala Asp Asp Thr Glu Ser Arg Phe Ala Thr Asn Thr Thr Leu Asp Val
   20 25 30
   Val Asn Leu Ile Ser Met Ala
   35
<210> 40
   <211> 39
   <212> PRT
   <213> Artificial
<220>
   <223> artificial
<400> 40
   Gln Pro Ile Asp Asp Thr Glu Ser Asn Thr Thr Ser Val Asn Leu Met
   1 5 10 15
   Ala Asn Thr Thr Glu Ser Arg Phe Ala Thr Asn Thr Thr Leu Asp Val
   20 25 30
   Val Asn Leu Ile Ser Met Ala
   35
<210> 41
   <211> 43
   <212> PRT
   <213> Artificial
<220>
   <223> artificial
<400> 41
   Gln Pro Ile Asp Asn Thr Glu Ser Asn Thr Thr Ser Val Asn Leu Met
   1 5 10 15
   Ala Asp Asp Thr Glu Ser Arg Phe Ala Thr Asn Thr Thr Leu Ala Leu
   20 25 30
   Asp Val Val Asn Leu Ile Ser Met Ala Lys Arg
   35 40
<210> 42
   <211> 5
   <212> PRT
   <213> Artificial
<220>
   <223> artificial
<400> 42
   Ser Asp Asp Ala Lys
   1 5
<210> 43
   <211> 6
   <212> PRT
   <213> Artificial
<220>
   <223> artificial
<400> 43
   Glu Glu Ala Glu Pro Lys
   1 5
<210> 44
   <211> 47
   <212> DNA
   <213> Artificial
<220>
   <223> artificial
<220>
   <221> misc_feature
   <222> (26)..(26)
   <223> n is A or G
<220>
   <221> misc_feature
   <222> (27)..(27)
   <223> m is T, G or A
<400> 44
   gatctccatg gctaagagag aagaanmtga accaaagttc gttaacc 47
<210> 45
   <211> 27
   <212> DNA
   <213> Artificial
<220>
   <223> artificial
<400> 45
   ttaatcttag tttctagagc ctgcggg 27
<210> 46
   <211> 5
   <212> PRT
   <213> Artificial
<220>
   <223> artificial
<400> 46
   Ser Asp Asp Ala Arg
   1 5
<210> 47
   <211> 42
   <212> PRT
   <213> Artificial
<220>
   <223> artificial
<220>
   <221> MISC_FEATURE
   <222> (1)..(1)
<220>
   <221> misc_feature
   <222> (1)..(1)
   <223> Xaa can be any naturally occurring amino acid
<220>
   <221> MISC_FEATURE
   <222> (23)..(23)
<220>
   <221> misc_feature
   <222> (23)..(23)
   <223> Xaa can be any naturally occurring amino acid
<220>
   <221> MISC FEATURE
   <222> (30)..(30)
<220>
   <221> misc_feature
   <222> (30)..(30)
   <223> Xaa can be any naturally occurring amino acid
<220>
   <221> misc_feature
   <222> (35)..(35)
   <223> Xaa can be any naturally occurring amino acid
<400> 47
   Xaa Pro Ile Asp Asp Thr Glu Ser Asn Thr Thr Ser Val Asn Leu Met
   1               5                   10                  15
   Ala Asp Asp Thr Glu Ser Xaa Phe Ala Thr Asn Thr Thr Xaa Leu Asp
               20                  25                  30
   Val Val Xaa Leu Ile Ser Met Ala Lys Arg
           35                  40
<210> 48
   <211> 4
   <212> PRT
   <213> Artificial
<220>
   <223> artificial
<400> 48
   Leu Ala Gly Gly
   1
<210> 49
   <211> 4
   <212> PRT
   <213> Artificial
<220>
   <223> artificial
<400> 49
   Ser Val Gly Gly
   1
<210> 50
   <211> 4
   <212> PRT
   <213> Artificial
<220>
   <223> artificial
<400> 50
   Asn Ser Gly Gly
   1
<210> 51
   <211> 5
   <212> PRT
   <213> Artificial
<220>
   <223> artificial
<400> 51
   Leu Ala Asp Gly Gly
   1 5
<210> 52
   <211> 4
   <212> PRT
   <213> Artificial
<220>
   <223> artifical
<400> 52
   Leu Ala Asp Asp
   1
<210> 53
   <211> 4
   <212> PRT
   <213> Artificial
<220>
   <223> artificial
<400> 53
   Leu Ala Gly Asp
   1
   1
   1
   1

## Description

### FIELD OF INVENTION

The present invention relates to polypeptides produced in yeast, a DNA construct comprising a DNA sequence encoding such polypeptides, vectors carrying such DNA fragments and yeast cells transformed with the vectors, as well as a process of producing heterologous proteins in yeast.

### BACKGROUND OF THE INVENTION

Yeast organisms produce a number of proteins synthesized intracellularly, but having a function outside the cell. Such extracelluar proteins are referred to as secreted proteins. These secreted proteins are expressed initially inside the cell in a precursor or a pre-form containing a pre-peptide sequence ensuring effective direction of the expressed product across the membrane of the endoplasmic reticulum (ER). The pre-peptide, normally named a signal peptide, is generally cleaved off from the desired product during translocation. Once entered in the secretory pathway, the protein is transported to the Golgi apparatus. From the Golgi the protein can follow different routes that lead to compartments such as the cell vacuole or the cell membrane, or it can be routed out of the cell to be secreted to the external medium (Pfeffer, S.R. and Rothman, J.E. Ann.Rev.Biochem. 56 (1987), 829-852.

Several approaches have been suggested for the expression and secretion in yeast of proteins heterologous to yeast. European Publication No. 0088632A describes a process by which proteins heterologous to yeast are expressed, processed and secreted by transforming a yeast organism with an expression vector harbouring DNA encoding the desired protein and a signal peptide, preparing a culture of the transformed organism, growing the culture and recovering the protein from the culture medium. The signal peptide may be the desired protein's own signal peptide, i.e. a heterologous signal peptide, or a hybrid of a homologous and a heterologous signal peptide.

A problem encountered with the use of signal peptides heterologous to yeast might be that the heterologous signal peptide does not ensure efficient translocation and/or cleavage after the signal peptide.

The Saccharomyces cerevisiae MFα1 (α-factor) is synthesized as a pre-pro form of 165 amino acids comprising a 19 amino acids long signal- or pre-peptide followed by a 64 amino acids long "leader" or pro-peptide, encompassing three N-linked glycosylation sites followed by (LysArg(Asp/Glu, Ala)₂₋₃α-factor)₄ (Kurjan, J. and Herskowitz, I. Cell 30 (1982), 933-943). The signal-leader part of the pre-pro MFα1 has been widely employed to obtain synthesis and secretion of heterologous proteins in S. cerevisiae.

Use of signal/leader peptides homologous to yeast is known from i.a. US Patent No. 4,546,082, European Publication Nos. 0116201A, 0123294A, 0123544A, 0163529A, 0123289A, European Patent No. 0100561B, and PCT Publication No. WO 95/02059.

In EP 0123289A utilization of the S. cerevisiae α-factor precursor is described whereas EP 0100561 describes the utilization of the Saccharomyces cerevisiae PHO5 signal and WO 95/02059 describes the utilization of YAP3 signal peptide for secretion of foreign proteins.

US Patent No. 4,546,082 and European Publication Nos. 0016201A, 0123294A, 0123544A and 0163529A describe processes by which the α-factor signal-leader from Saccharomyces cerevisiae (MFα1 or MFα2) is utilized in the secretion process of expressed heterologous proteins in yeast. Secretion and processing of the desired protein was demonstrated by fusing a DNA sequence encoding the S. cerevisiae MFα1 signal/leader peptide at the 5' end of the gene for the desired protein.

EP 0206783 discloses a system for the secretion of polypeptides from S. cerevisiae whereby the α-factor signal/leader sequence has been truncated to eliminate the four α-factor peptides present on the native sequence so as to leave the signal/leader peptide itself fused to a heterologous polypeptide via the α-factor processing site Lys-Arg-Glu-Ala-Glu-Ala. It is indicated that this construction leads to an efficient process for production of smaller peptides (less than 50 amino acids). For the secretion and processing of larger polypeptides, the native α-factor leader sequence has been truncated to leave one or two α-factor peptides between the leader peptide and the polypeptide.

A number of secreted proteins are routed so that the precursor is exposed to a proteolytic processing system which can cleave the peptide bond at the carboxy end of two consecutive basic amino acids. This enzymatic activity is in S. cerevisiae encoded by the KEX 2 gene (Julius, D.A. et al., Cell 37 (1984b), 1075). Processing of the product by the KEX 2 protease is needed for the secretion of active S. cerevisiae mating factor α1 (MFα1 or α-factor) but is not involved in the secretion of active S. cerevisiae mating factor a.

Secretion and correct processing of a polypeptide intended to be secreted is obtained in some cases when culturing a yeast organism which is transformed with a vector constructed as indicated in the references given above. In many cases, however, the level of secretion is very low or there is no secretion, or the proteolytic processing may be incorrect or incomplete. As described in PCT Publication No. WO 90/10075 this is believed to be ascribable, to some extent, to an insufficient exposure of the processing site present between the C-terminal end of the leader peptide and the N-terminal end of the heterologous protein so as to render it inaccessible, or less accessible, to proteolytic cleavage, i.a. cleavage by the KEX 2 protease.

WO 90/10075 describes a yeast expression system with improved processing of a heterologous polypeptide obtained by providing certain modifications near the processing site at the C-terminal end of the leader peptide and/or the N-terminal end of a heterologous polypeptide fused to the leader peptide. Thus, it is possible to obtain a higher yield of the correctly processed protein than is obtainable with unmodified leader peptide-heterologous polypeptide constructions.

Furthermore, WO 95/35384 describes DNA constructs encoding polypeptides having modifications of the N-terminal end of the heterologous polypeptide designed as extensions which can be cleaved off by yeast proteases before purification from the culture media or by *in vitro* proteolysis during or subsequent to purification of the product from the culture medium. Said polypeptides are of the formula signal peptide-leader peptide-X¹-X²-X³-X⁴-X⁵-X⁶-X⁷-heterologous protein,
wherein X¹-X² defines a dibasic yeast protease processing site, such as Lys-Arg, and
wherein the sequence X³-X⁴-X⁵-X⁶-X⁷ does not comprise EEGEPK.

### SUMMARY OF THE INVENTION

The present invention describes an improvement of modifications of the N-terminal end of the heterologous polypeptide designed as extensions which can be cleaved off by in vitro proteolysis during or subsequent to purification of the product from the culture media resulting in higher yields of a homogeneous heterologous protein product.

The present invention relates to a DNA construct encoding a polypeptide sequence having the following structure
SP-LP-EEGEPK-*polypeptide*
wherein SP is a DNA sequence encoding a signal peptide,
LP is a DNA sequence encoding a leader peptide, and
EEGEPK is an amino acid sequence located as an N-terminal extension of the following *polypeptide* which is a protein or other polypeptide to be produced.

The conventional one-letter code for amino acids in accordance with rules approved (1974) by the IUPAC-IUB Commission of Biochemical Nomenclature is used throughout this description and the claims.

It is, without being bound by any specific theory as to the mechanism of action of the N-terminal extensions of the invention, surprising, that the location of a glycine (G) in the N-terminal extension compared to the repeated GluAla of the αfactor leader results in improved heterologous protein yield reflecting a possible improved translocation and/or secretion, since glycine with only a hydrogen atom as a side chain can adopt a much wider range of conformations than other amino acid residues, thus allowing unusual main chain conformations, and a possible more unstable precursor polypeptide and secretion process.

The sequence EEGEPK forms an extension at the N-terminal of the heterologous polypeptide. This extension not only increases the fermentation yield but is protected against dipeptidyl aminopeptidase (DPAP A) processing, resulting in a homogenous N-terminal of the polypeptide. The extension is constructed in such a way that it is resistant to proteolytic cleavage during fermentation so that the N-terminally extended heterologous protein product can be purified from the culture media for subsequent *in vitro* proteolytic maturation, e.g. by a protease specific for basic amino acids, such as trypsin or *Achromobacter lyticus* protease I. The desired *in vitro* removal of the N-terminal extension EEGEPK is readily achieved by either trypsin or Achromobacter lyticus protease I presumably due to flexibility of the N-terminal extension peptide resulting in an improved yield of the matured heterologous protein.

In the present context, the term "signal peptide" is understood to mean a pre-peptide which is present as an N-terminal sequence on the precursor form of an extracellular protein expressed in yeast. The function of the signal peptide is to allow the heterologous protein to be secreted to enter the endoplasmic reticulum. The signal peptide is normally cleaved off in the course of this process. The signal peptide may be heterologous or homologous to the yeast organism producing the protein. A preferred signal peptide in this invention is yeast aspartic protease 3 (YAP3) signal peptide. YAP 3 has been cloned and characterised by Egel-Mitani, M. et al., YEAST, 6, p. 127-137, 1990.

The expression "leader peptide" is understood to indicate a polypeptide sequence whose function is to allow the heterologous protein to be secreted to be directed from the endoplasmic reticulum to the Golgi apparatus and further to a secretory vesicle for secretion into the medium, (i.e. exportation of the expressed protein or polypeptide across the cell wall or at least through the cellular membrane into the periplasmic space of the cell). Preferably the leader peptide used in the present invention is selected from the following group of leader peptides
identified as SEQ ID No. 1 through 28 disclosed in WO 95/34666 and all C-terminally followed by a Lys-Arg sequence and any functional analogue thereof, and more preferably the leader peptide has an amino acid sequence of 43 or more amino acids, such as the leader peptide (LA19):
identified as SEQ ID No. 67 in WO 95/34666 and which includes the C-terminal LysArg processing site. In the DNA construct of the present invention the leader peptide preferably contains an endopeptidase processing site at the C-terminal end, such as a Lys-Arg sequence.

Even more preferred leader peptides encoded by the DNA constructs of the invention are

The expression "heterologous protein" is intended to indicate a protein or polypeptide which is not produced by the host yeast organism in nature.

In a still further aspect, the invention relates to a process for producing a heterologous protein in yeast, comprising cuttivating the transformed yeast strain in a suitable medium to obtain expression and secretion of the heterologous protein, after which the protein is isolated from the medium.

### DETAILED DESCRIPTION OF THE INVENTION

The N-terminally extended heterologous protein produced by the method of the invention may be any protein which may advantageously be produced in yeast. Examples of such proteins are aprotinin, tissue factor pathway inhibitor or other protease inhibitors, and insulin or insulin precursors, insulin analogues, insulin-like growth factors, such as IGF I and IGF II, human or bovine growth hormone, interleukin, tissue plasminogen activator, glucagon, glucagon-like peptide-1 (GLP 1), glucagon-like peptide-2 (GLP 2), GRPP, Factor VII, Factor VIII, Factor XIII, platelet-derived growth factor, enzymes, such as lipases. Preferred proteins are precursors of insulin and insulin like growth medium to obtain expression and secretion of the heterologous protein, after which the protein is isolated from the medium.

### DETAILED DESCRIPTION OF THE INVENTION

The N-terminally extended heterologous protein produced by the method of the invention may be any protein which may advantageously be produced in yeast. Examples of such proteins are aprotinin, tissue factor pathway inhibitor or other protease inhibitors, and insulin or insulin precursors, insulin analogues, insulin-like growth factors, such as IGF I and IGF II, human or bovine growth hormone, interleukin, tissue plasminogen activator, glucagon, glucagon-like peptide-1 (GLP 1), glucagon-like peptide-2 (GLP 2), GRPP, Factor VII, Factor VIII, Factor XIII, platelet-derived growth factor, enzymes, such as lipases, or a functional analogue of any one of these proteins. Preferred proteins are precursors of insulin and insulin like growth factors, and peptides of the proglucagon family, such as glucagon, GLP 1, GLP 2, and GRPP, including truncated forms, such as GLP-1(1-45), GLP-1(1-39), GLP-1(1-38), GLP-1(1-37), GLP-1(1-36), GLP-1(1-35), GLP-1(1-34), GLP-1(7-45), GLP-1(7-39), GLP-1 (7-38), GLP-1(7-37). GLP-1 (7-36), GLP-1(7-35), and GLP-1(7-34).

In the present context, the term "functional analogue" is meant to indicate a polypeptide with a similar function as the native protein (this is intended to be understood as relating to the nature rather than the level of biological activity of the native protein). The polypeptide may be structurally similar to the native protein and may be derived from the native protein by addition of one or more amino acids to either or both the C- and N-terminal end of the native protein, substitution of one or more amino acids at one or a number of different sites in the native amino acid sequence, deletion of one or more amino acids at either or both ends of the native protein or at one or several sites in the amino acid sequence, or insertion of one or more amino acids at one or more sites in the native amino acid sequence. Such modifications are well known for several of the proteins mentioned above.

The precursors of insulin, including proinsulin as well as precursors having a truncated and/or modified C-peptide or completely lacking a C-peptide, precursors of insulin analogues, and insulin related peptides, such as insulin like growth factors, may be of human origin or from other animals and recombinant or semisynthetic sources. The cDNA used for expression of the precursors of insulin, precursors of insulin analogues, or insulin related peptides in the method of the invention include codon optimised forms for expression in yeast.

By "a precursor of insulin" or "a precursor an insulin analogue" is to be understood a single-chain polypeptide which by one or more subsequent chemical and/or enzymatical processes can be converted to a two-chain insulin or insulin analogue molecule having the correct establishment of the three disulphide bridges as found in natural human insulin. Preferred insulin precursors are B(1-29)-A(1-21) designated MI1; B(1-29)-Ala-Ala-Lys-A(1-21) designated MI3 (as described in e.g. EP 163 529); B(1-27-Asp-Lys)-Ala-Ala-Lys-A(1-21) designated X14 (as described in e.g. PCT publication No. 95/00550); B(1-27-Asp-Lys)-A(1-21); B(1-27-Asp-Lys)-Ser-Asp-Asp-Ala-Lys-A(1-21); B(1-29)-Ala-Ala-Arg-A(1-21) (as described in e.g. PCT Publication No. 95/07931); B(1-29)-Ser-Asp-Asp-Ala-Lys-A(1-21) designated MI5 and B(1-29)-Ser-Asp-Asp-Ala-Arg-A(1-21), and more preferably B(1-29)-A(1-21), designated MI1; B(1-29)-Ala-Ala-Lys-A(1-21), which designated MI3 and B(1-29)-Ser-Asp-Asp-Ala-Lys-A(1-21), designated MI 5.

Examples of insulins or insulin analogues which can be produced in this way are human insulin, preferably des(B30) human insulin, porcine insulin; and insulin analogues wherein at least one Lys or Arg is present, preferably insulin analogues wherein Phe^{B1} has been deleted, insulin analogues wherein the A-chain and/or the B-chain have an N-terminal extension and insulin analogues wherein the A-chain and/or the B-chain have a C-terminal extension. Other preferred insulin analogues are such wherein one or more of the amino acid residues, preferably one, two, or three of them, have been substituted by another codable amino acid residue. Thus in position A21 a parent insulin may instead of Asn have an amino acid residue selected from the group comprising Ala, Gln, Glu, Gly, His, lle, Leu, Met, Ser, Thr, Trp, Tyr or Val, in particular an amino acid residue selected from the group comprising Gly, Ala, Ser, and Thr. The insulin analogues may also be modified by a combination of the changes outlined above. Likewise, in position B28 a parent insulin may instead of Pro have an amino acid residue selected from the group comprising Asp and Lys, preferably Asp, and in position B29 a parent insulin may instead of Lys have the amino acid Pro. The expression "a codable amino acid residue" as used herein designates an amino acid residue which can be coded for by the genetic code, i. e. a triplet ("codon") of nucleotides.

The DNA construct of the invention encoding the polypeptide of the invention may be prepared synthetically by established standard methods, e.g. the phosphoamidite method described by S.L. Beaucage and M.H. Caruthers, Tetrahedron Letters 22, 1981, pp. 1859-1869, or the method described by Matthes et al., EMBO Journal 3, 1984, pp. 801-805. According to the phosphoamidite method, oligonucleotides are synthesized, e.g. in an automatic DNA synthesizer, purified, duplexed and ligated to form the synthetic DNA construct. A currently preferred way of preparing the DNA construct is by polymerase chain reaction (PCR), e.g. as described in Sambrook et al., Molecular Cloning: A Laboratory Manual, Cold Spring Harbor, NY, 1989).

The DNA construct of the invention may also be of genomic or cDNA origin, for instance obtained by preparing a genomic or cDNA library and screening for DNA sequences coding for all or part of the polypeptide or the invention by hybridization using synthetic oligonucleotide probes in accordance with standard techniques (cf. Sambrook et al., Molecular Cloning: A Laboratory Manual, Cold Spring Harbor, 1989). In this case, a genomic or cDNA sequence encoding a signal and leader peptide may be joined to a genomic or cDNA sequence encoding the heterologous protein, after which the DNA sequence may be modified at a site corresponding to the amino acid sequence EEGEPK of the polypeptide, by inserting synthetic oligonucleotides encoding the desired amino acid sequence for homologous recombination in accordance with well-known procedures or preferably generating the desired sequence by PCR using suitable oligonucleotides.

Finally, the DNA construct may be of mixed synthetic and genomic, mixed synthetic and cDNA or mixed genomic and cDNA origin prepared by annealing fragments of synthetic, genomic or cDNA origin (as appropriate), the fragments corresponding to various parts of the entire DNA construct, in accordance with standard techniques. Thus, it may be envisaged that the DNA sequence encoding the heterologous protein may be of genomic or cDNA origin, while the sequence encoding the signal and leader peptide as well as the sequence encoding the N-terminal extension EEGEPK may be prepared synthetically.

In a further aspect, the invention relates to a recombinant expression vector which is capable of replicating in yeast and which carries a DNA construct encoding the above-defined polypeptide. The recombinant expression vector may be any vector which is capable of replicating in yeast organisms. In the vector, the DNA sequence encoding the polypeptide of the invention should be operably connected to a suitable promoter sequence. The promoter may be any DNA sequence which shows transscriptional activity in yeast and may be derived from genes encoding proteins either homologous or heterologous to yeast. The promoter is preferably derived from a gene encoding a protein homologous to yeast. Examples of suitable promoters are the Saccharomyces cerevisiae Mα1, TPI, ADH or PGK promoters.

The DNA sequence encoding the polypeptide of the invention may also be operably connected to a suitable terminator, e.g. the TPI terminator (cf. T. Alber and G. Kawasaki, J. Mol, Appl, Genet. 1, 1982, pp. 419-434).

The recombinant expression vector of the invention comprises a DNA sequence enabling the vector to replicate in yeast. Examples of such sequences are the yeast plasmid 2µ replication genes REP 1-3 and origin of replication. The vector may also comprise a selectable marker, e.g. the Schizosaccharomyces pompe TPI gene as described by P.R. Russell, Gene 40, 1985, pp. 125-130.

The procedures used to ligate the DNA sequences coding for the polypeptide of the invention, the promoter and the terminator, respectively, and to insert them into suitable yeast vectors containing the information necessary for yeast replication, are well known to persons skilled in the art (cf., for instance, Sambrook et al., *op.cit*.). It will be understood that the vector may be constructed either by first preparing a DNA construct containing the entire DNA sequence coding for the polypeptide of the invention and subsequently inserting this fragment into a suitable expression vector, or by sequentially inserting DNA fragments containing genetic information for the individual elements (such as the signal, leader or heterologous protein) followed by ligation.

The yeast organism used in the process of the invention may be any suitable yeast organism which, on cultivation, produces large amounts of the heterologous protein or polypeptide in question. Examples of suitable yeast organisms may be strains selected from the yeast species Saccharomyces cerevisiae, Saccharomyces kluyveri, Schizosaccharomyces pombe, Sacchoromyces uvarum, Kluyveromyces lactis, Hansenula polymorpha, Pichia pastoris, Pichia methanolica, Pichia kluyveri, Yarrowia lipolytica, Candida sp., Candida utilis, Candida cacaoi, Geotrichum sp., and Geotrichum fermentans. It is considered obvious for the skilled person in the art to select any other fungal cell, such as cells of the genus Aspergillus, as the host organism.

The transformation of the yeast cells may for instance be effected by protoplast formation followed by transformation in a manner known *per se*. The medium used to cultivate the cells may be any conventional medium suitable for growing yeast organisms. The secreted heterologous protein, a significant proportion of which will be present in the medium in correctly processed form, may be recovered from the medium by conventional precedures including separating the yeast cells from the medium by centrifugation or filtration, precipitating the proteinaceous components of the supernatant or filtrate by means of a salt, e.g. ammonium sulphate, followed by purification by a variety of chromatographic procedures, e.g. ion exchange chromatography, affinity chromatography, or the like.

After secretion to the culture medium, the protein may be subjected to various precedures to remove the sequence EEGEPK.

The extension is found to be stably attached to the heterologous protein during fermentation, protecting the N-terminal of the heterologous protein against the proteolytic activity of yeast proteases such as DPAP. The presence of an N-terminal extension on the heterologous protein may also serve as a protection of the N-terminal amino group of the heterologous protein during chemical processing of the protein, i.e. it may serve as a substitute for a BOC (t-butyl-oxycarbonyl) or similar protecting group. In such cases the amino acid sequence EEGEPK may be removed from the recovered heterologous protein by means of a proteolytic enzyme which is specific for a basic amino acid (i.e. K (Lys)) so that the terminal extension is cleaved off at the K. Examples of such proteolytic enzymes are trypsin or *Achromobacter lyticus* protease I.

The present invention is described in further detain in the following examples which are not in any way intended to limit the scope of the invention as claimed.

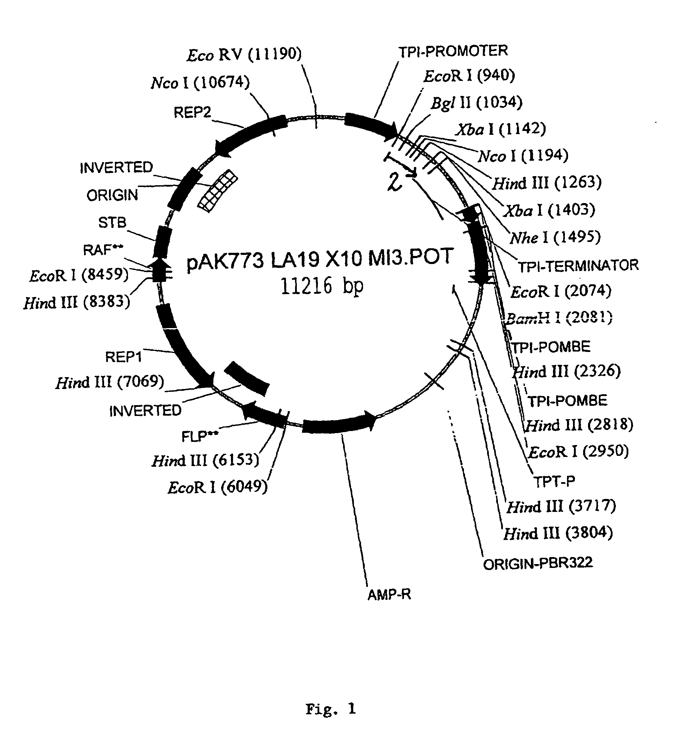

The invention is described with reference to the drawings, wherein
- Fig. 1: shows the expression plasmid pAK773 containing genes expressing the N-terminally extended polypeptides of the invention. In Fig. 1 the following symbols are used: TPI-PROMOTER: Denotes the TPI gene promoter sequence from *S*. *cerevisiae.*
2: Denotes the region encoding a signal/leader peptide (e.g. from the YAP3 signal peptide and LA19 leader peptide in conjunction with the EEGEPK N-terminally extended MI3 insulin precursor.
TPI-TERMINATOR: Denotes TPI gene terminator sequence of *S. cerevisiae.*
TPI-POMBE: Denotes TPI gene from *S. pombe.*
Origin: Denotes a sequence from *S*. *cerevisiae* 2 µ plasmid including its origin of DNA replication in *S*. *cerevisiae.*
AMP-R: Sequence from pBR322 /pUC13 including the ampicillin resistance gene and an origin of DNA replication in *E. coli.*
- Fig. 2: shows the DNA and amino acid sequences in pAK773 encoding the YAP3 signal peptide (amino acid No. 1 through 21). LA19 leader peptide (amino acid No. 22 through 64), N-terminal extension EEGEPK (amino acid No. 65 through 70), MI3 insulin precursor B_{chain}(1-29)-Ala-Ala-Lys-A_{chain}(1-21) (amino acid No. 71 through 123).
- Fig. 3: shows the expression plasmid pAK729 containing genes expressing the N-terminally extended polypeptides of the invention, and the following symbols are used:
TPI-PROMOTER: Denotes the TPI gene promoter sequence from S. cerevisiae.
2: Denotes the region encoding a signal/leader peptide (e.g. from the YAP3 signal peptide and LA19 leader peptide in conjunction with the EEAEPK N-terminally extended M13 insulin precursor.
TPI-TERMINATOR: Denotes TPI gene terminator sequence of S. cerevisiae.
TPI-POMBE: Denotes TPI gene from S. pombe.
Origin: Denotes a sequence from S. cerevisiae 2 µ plasmid including its origin of DNA replication in S. cerevisiae.
AMP-R: Sequence from pBR322 /pUC13 including the ampicillin resistance gene and an origin of DNA replication in E. coli.

### Example

### Construction of the yeast strain yAK773 expressing the EEGEPK-MI3 insulin precursor

A synthetic gene coding for the N-terminal extension of N-terminally extended insulin precursor EEGEPK-MI3 was constructed using the Polymerase Chain Reaction (PCR).

The following 2 oligonucleotides were synthesized:
wherein X is A or G and Y is T, A, or G, and

Oligonucleotides were synthesized using an automatic DNA synthesizer (applied Biosystems model 380A) using phosphoamidite chemistry and commercially available reagents (Beaucage, S.L. and Caruthers, M.H., Tetrahedron letters 22 (1981) 1859-1869).

The following PCR was performed using the Expand High Fidelity Enzyme mix (Boehringer Mannheim GmbH, Sandhoefter Strasse 116, Mannheim, Germany) according to the manufacturers instructions and The PCR mix was overlayed with 100 ul mineral oil (Sigma Chemical Co., St. Louis MO, USA)

### PCR

5 µl oligonucleotide #724 (total 100 pmol)
5 µl oligonucleotide #2371 (total 100 pmol)
10 µl 10X PCR buffer
8 µl dNTP mix
0.75 µl Expand High Fidelity Enzyme mix
0.5 µl pAK729 plasmid as template (0.2 µg DNA)
70,75 µl distilled water

A total of 18 cycles were performed, one cycle was 94°C for 45 se.; 37°C for 1 min; 72°C for 1.5 min. The PCR mixture was then loaded onto a 2.5% agarose gel and electroforese was performed using standard techniques (Sambrook J, Fritsch El and Maniatis T, Molecular cloning, Cold Spring harbour laboratory press, 1989). The resulting DNA fragment was cut out of the agarose gel and isolated by the Gene Clean Kit (Bio 101 inc., PO BOX 2284, La Jolla, CA 92038, USA) according to the manufacturer's instructions.

The purified PCR DNA fragment was dissolved in 14 µl of water and restriction endonucleases buffer and cut with the restriction endonucleases Ncol and Xbal according to standard techniques (Sambrook J, Fritsch EF and Maniatis T, Molecular cloning, Cold spring Harbour laboratory press, 1989). The Ncol-Xbal DNA fragment on 209 nucleotide base pairs was subjected to agarose electroforese and purified using The Gene Clean Kit as described.

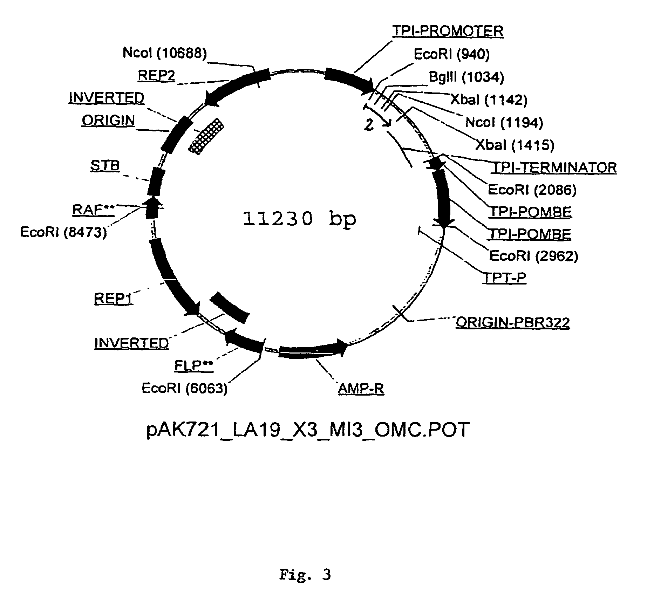

The plasmid pAK721 (see Fig. 3) was cut with the restriction endonucleases Bglll and Xbal and the vector fragment of 10849 nucleotide base pairs isolated using The Gene Clean Kit as described.

The plasmid pAK721 was cut with the restriction endonucleases Bglll and Ncol and the DNA fragment of 160 nucleotide base pairs isolated using The Gene Clean Kit as described.

The three DNA fragments was ligated together using T4 DNA ligase and standard conditions (Sambrook J, Fritsch EF and Maniatis T, Molecular cloning, Cold spring Harbour laboratory press, 1989). The ligation mix was then transformed into a competent *E*. *coli* strain (R-, M+) followed by selection with ampicillin resistance.

Plasmid from the resulting *E*. *coli* was isolated using standard techniques (Sambrook J, Fritsch EF and Maniatis T, Molecular cloning, Cold spring Harbour laboratory press, 1989), and checked for insert with appropriate restriction endonucleases i.e. Bglll and Nhel. The selected plasmid was shown by DNA sequence analyse (Sequenase, U.S. Biochemical Corp., USA) to encode the correct DNA sequence for the EEGEPK-MI3 insulin precursor DNA and to be inserted after the DNA encoding the synthetic LA19 leader DNA.

The plasmid was named pAK773.

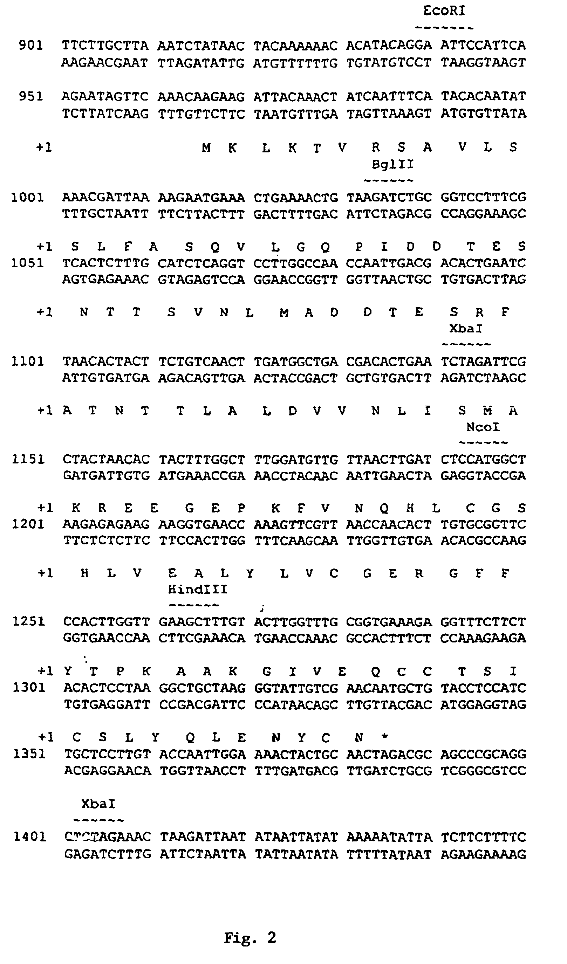

The DNA sequence encoding the YAP3 signal peptide-LA19 leader EEGEPK-M13 insulin precursor complex are shown in Fig. 2.

The plasmid pAK773 was transformed into S. cerevisiae strain MT663 as described in PCT/DK95/00250 and the resulting strain was named yAK773.

The yeast expression plasmid pAK773 is of the C-POT type (see Fig. 1) and is similar to those described in WO EP 171 142, which contain the *Schizosaccharomyces pombe* triose phosphate isomerase gene (POT) for plasmid selection and stabilisation in S. cerevisiae. pAK773 also contain the S. cerevisiae triose phosphate isomerase promoter and terminator. The promoter and terminator are similar to those described in the plasmid pKFN1003 (described in WO 90/100075) as are all sequences in plasmid except the sequence between the EcoRlXbal fragment encoding the YAP3 signal peptide-LA19 leader peptide-EEGEPK-Ml3 insulin precursor.

## Claims

1. A DNA construct encoding a polypeptide sequence having the following structure SP-LP-EEGEPK-*polypeptide*
wherein SP is a DNA sequence encoding a signal peptide,
LP is a DNA sequence encoding a leader peptide, and
EEGEPK is an amino acid sequence located as an N-terminal extension of the following *polypeptide* which is a protein or other polypeptide to be produced.

2. A DNA construct according to the preceding claim, wherein SP is a PNA sequence selected from the group of DNA sequences encoding the S. cerevisiae α-factor signal peptide, the signal peptide of mouse salivary amylase, the yeast carboxypeptidease signal peptide, the yeast aspartic protease 3 (YAP3) signal peptide, or the yeast BAR1 signal peptide.

3. A DNA construct according to any one of the preceding claims wherein SP is a DNA sequence encoding the yeast aspartic protease 3 (YAP3) signal peptide.

4. A DNA construct according to any one of the preceding claims wherein LP is a DNA sequence encoding a synthetic leader peptide of 43 amino acids or more.

5. A DNA construct according to any one of the preceding claims wherein LP is a DNA sequence encoding a synthetic leader peptide of the formula
wherein X1 is at least one amino acid selected from the group consisting of A, D, E, F, G, H, I, K L, M. N, P, Q, R, S, T, V, and Y;
X₂ is a peptide bond or one or more amino acids selected from the group consisting of A, D, E, F, G, H, I, K, L, M, N, P. Q, R, S, T, V, Y;
X₃ is from 1 to 10 amino acids selected from the group consisting of A, D, E, F, G, H, I, K, L, M. N, P, Q, R, S, T, V, Y; and
X₄ is a peptide bond or one or two amino acids selected from the group consisting of A, D, E, F, G, H, I, K, L, M, N, P, Q, R, S, T, V, Y.

6. A DNA construct according to claim 5 wherein X1 is an amino acid selected from the group consisting of Q and S.

7. A DNA construct according to claim 5 wherein X2 is one amino acid selected from the group consisting of R and A.

8. A DNA construct according to claim 5 wherein X3 is a sequence of from two to five amino acids selected from the group consisting of LA, LAGG, SVGG, NSGG, LADGG, LADD, and LAGD.

9. A DNA construct according to claim 5 wherein X4 is one amino acid selected from the group consisting of N and G.

10. A DNA construct according to any claims1-5 wherein LP is a DNA sequence encoding a synthetic leader peptide selected from the group consisting of or

11. A DNA construct according to any one of claims 1, 2, 3 or 4, wherein LP is a DNA sequence encoding the synthetic leader peptide (LA19):

12. A DNA construct according to any one of the preceding claims, wherein the "polypeptide" is a heterologous protein.

13. A DNA construct according to claim 12 wherein the heterologous protein is selected from the group consisting of aprotinin, tissue factor pathway inhibitor or other protease inhibitors, insulin-like growth factor I or II, human or bovine growth hormone, interieukin, tissue plasminogen activator, proglucagon and proglucagon derived polypeptides, glucagon, glucagon like peptide-1, glucagon-like peptide 2, GRPP, Factor VII, Factor VIII, Factor XIII, platelet derived growth factor, enzymes, insulin or an insulin precursor.

14. A DNA construct according to the preceding claim wherein the proglucagon derived polypeptides are selected from the group consisting of GLP-1 (1-45), GLP-1(1-39), GLP-1(1-38), GLP-1(1-37), GLP-1(1-36), GLP-1(1-35), GLP-1(1-34), GLP-1(7-45), GLP-1(7-39), GLP-1(7-38), GLP-1(7-37), GLP-1(7-36), GLP-1(7-35), and GLP-1(7-34).

15. A DNA construct according to claim 13, wherein the heterologous protein is insulin or an insulin precursor, preferably selected from the group consisting of B(1-29)-A(1-21); B(1-27-Asp-Lys)-Ala-Ala-Lys A(1-21); B(1-29)-Ala-Ala-Lys-A(1-21); and B(1-29)-Ser-Asp-Asp-Ala-Lys-A(1-21).

16. The DNA construct described in Fig. 2 herein.

17. A polypeptide having the sequence EEGEPK-*polypeptide*, wherein EEGEPK is the N-terminal extension of the following *polypeptide* which is a protein or other polypeptide to be produced.

18. A polypeptide according to claim 17 wherein *polypeptide* is selected from the group consisting of aprotinin, tissue factor pathway inhibitor or other protease inhibitors, insulin-like growth factor I or II, human or bovine growth hormone, interieukin, tissue plasminogen activator, proglucagon and proglucagon derived polypeptides, glucagon, glucagon-like peptide-1, glucagon-like peptide 2, GRPP, Factor VII, Factor VIII, Factor XIII, platelet derived growth factor, enzymes, insulin or an insulin precursor.

19. A polypeptide according to claim 18 wherein the proglucagon derived polypeptides are selected from the group consisting of GLP-1(1-45), GLP-1(1-39), GLP-1(1-38), GLP-1(1-37), GLP-1(1-36), GLP-1(1-35), GLP-1(1-34), GLP-1(7-45), GLP-1(7-39), GLP-1(7-38), GLP-1(7-37), GLP-1(7-36), GLP-1(7-35), and GLP-1(7-34).

20. A polypeptide according to claim 18 wherein the "polypeptide" is insulin or an insulin precursor selected from the group consisting of B(1-29)-A(1-21); B(1-27-Asp-Lys)-Ala-Ala-Lys-A(1-21); B(1-29)-Ala-Ala-Lys-A(1-21); and B(1-29)-Ser-Asp-Asp-Ala-Lys-A(1-21).

21. A recombinant expression vector which is capable of replicating in yeast and which carries a DNA construct according to any one of the preceding claims 1-16 operably connected to a promoter sequence, preferably the TPI promoter sequence, as well as a terminator sequence, preferably the TPI terminator sequence, which are suitable for expression in yeast.

22. A yeast cell which is capable of expressing a heterologous protein and which is transformed with a vector according to claim 21.

23. A yeast cell according to claim 22 and which belongs to any one of the species Saccharomyces cerevisiae, Saccharomyces kluyveri. Schizosaccharomyces pombe, Hansenula polymorpha, and Yarrowia lipotytica.

24. A process for producing a heterologous protein, comprising cultivation a yeast cell according to any one of claims 22 or 23 in a suitable medium to obtain expression and secretion of the heterologous protein, after which the protein is isolated from the culture medium.

25. A process according to claim 24, wherein the amino acid sequence EEGEPK is removed from the recovered heterologous protein by a process involving treatment with a proteolytic enzyme which is specific for a basic amino acid.

26. A process according to claim 25, wherein the proteolytic enzyme is selected from the group consisting of trypsin and *Achromobacter lyticus* protease I.

## Patentansprüche

1. DNA-Konstrukt, das eine Polypeptidsequenz mit der folgenden Struktur SP-LP-EEGEPK-*Polypeptid* kodiert,
wobei SP eine ein Signalpeptid kodierende DNA-Sequenz ist,
LP eine ein Leaderpeptid kodierende DNA-Sequenz ist und
EEGEPK eine Aminosäuresequenz ist, die als N-terminale Verlängerung des folgenden *Polypeptids*, bei welchem es sich um ein herzustellendes Protein oder ein anderes Polypeptid handelt, lokalisiert ist.

2. DNA-Konstrukt nach dem vorangehenden Anspruch, wobei SP eine DNA-Sequenz ist, die aus der Gruppe von DNA-Sequenzen ausgewählt ist, die das Signalpeptid des α-Faktors von *S*. *cerevisiae,* das Signalpeptid der Mäusespeichelamylase, das Signalpeptid der Hefecarboxypeptidase, das Signalpeptid der Hefeaspartamprotease 3 (YAP3) oder das Signalpeptid der Hefe-BAR1 kodieren.

3. DNA-Konstrukt nach einem der vorangehenden Ansprüche, wobei SP eine DNA-Sequenz ist, die das Signalpeptid der Hefeaspartamprotease 3 (YAP3) kodiert.

4. DNA-Konstrukt nach einem der vorangehenden Ansprüche, wobei LP eine DNA-Sequenz ist, die ein synthetisches Leaderpeptid mit 43 Aminosäuren oder mehr kodiert.

5. DNA-Konstrukt nach einem der vorangehenden Ansprüche, wobei LP eine DNA-Sequenz ist, die ein synthetisches Leaderpeptid der Formel
kodiert, wobei X1 mindestens eine Aminosäure ist, ausgewählt aus der Gruppe, bestehend aus A, D, E, F, G, H, I, K, L, M, N, P, Q, R, S, T, V und Y;
X2 eine Peptidbindung oder eine oder mehrere Aminosäuren ist, ausgewählt aus der Gruppe, bestehend aus A, D, E, F, G, H, I, K, L, M, N, P, Q, R, S, T, V, Y;
X3 1 bis 10 Aminosäuren ist, ausgewählt aus der Gruppe, bestehend aus A, D, E, F, G, H, I, K, L, M, N, P, Q, R, S, T, V, Y; und
X4 eine Peptidbindung oder eine oder zwei Aminosäuren ist, ausgewählt aus der Gruppe, bestehend aus A, D, E, F, G, H, I, K, L, M, N, P, Q, R, S, T, V, Y.

6. DNA-Konstrukt nach Anspruch 5, wobei X1 eine Aminosäure ist, ausgewählt aus der Gruppe bestehend aus Q und S.

7. DNA-Konstrukt nach Anspruch 5, wobei X2 eine Aminosäure ist, ausgewählt aus der Gruppe bestehend aus R und A.

8. DNA-Konstrukt nach Anspruch 5, wobei X3 eine Sequenz aus zwei bis fünf Aminosäuren ist, ausgewählt aus der Gruppe bestehend aus LA. LAGG, SVGG, NSGG, LADGG, LADD und LAGD.

9. DNA-Konstrukt nach Anspruch 5, wobei X4 eine Aminosäure ist, ausgewählt aus der Gruppe bestehend aus N und G.

10. DNA-Konstrukt nach einem der Ansprüche 1-5, wobei LP eine DNA-Sequenz ist, die ein synthetisches Leaderpeptid, ausgewählt aus der Gruppe, bestehend aus
oder
kodiert.

11. DNA-Konstrukt nach einem der Ansprüche 1, 2, 3 oder 4, wobei LP eine DNA-Sequenz ist, die das synthetische Leaderpeptid (LA19) kodiert:

12. DNA-Konstrukt nach einem der vorangehenden Ansprüche, wobei das *Polypeptid* ein heterologes Polypeptid ist.

13. DNA-Konstrukt nach Anspruch 12, wobei das heterolologe Protein ausgewählt ist aus der Gruppe, bestehend aus Aprotinin, Gewebefaktorweghemmer oder anderen Proteasehemmern, insulinartigem Wachstumsfaktor I oder 11, Human- oder Rinderwachstumshormon, Interleukin, Gewebeplasminogenaktivator, Proglucagon und von Proglucagon abgeleiteten Polypeptiden, Glucagon, glucagonartigem Peptid-1, glucagonartigem Peptod-2, GRPP, Faktor VII, Faktor VIII, Faktor XIII, von Plättchen abgeleitetem Wachstumsfaktor, Enzymen, Insulin oder einem Insulinvorläufer.

14. DNA-Konstrukt nach dem vorangehenden Anspruch, wobei die von Proglucagon abgeleiteten Polypeptide ausgewählt sind aus der Gruppe, bestehend aus GLP-1(1-45), GLP-1(1-39), GLP-1(1-38), GLP-1(1-37), GLP-1(1-36), GLP-1(1-35), GLP-1(1-34), GLP-1(7-45), GLP-1(7-39), GLP-1(7-38), GLP-1(7-37), GLP-1(7-36), GLP-1(7-35) und GLP-1(7-34).

15. DNA-Konstrukt nach Anspruch 13, wobei das heterologe Protein Insulin oder ein Insulinvorläufer, vorzugsweise ausgewählt aus der Gruppe, bestehend aus B(1-29)-A(1-21); B(1-27-Asp-Lys)-Ala-Ala-Lys-A(1-21); B(1-29)-Ala-Ala-Lys-A(1-21); und B(1-29)-Ser-Asp-Asp-Ala-Lys-A(1-21)
ist.

16. DNA-Konstrukt wie hier in Fig. 2 beschrieben.

17. Polypeptid mit der Sequenz EEGEPK-*Polypeptid*, wobei EEGEPK die N-terminale Verlängerung des folgenden *Polypeptids*, bei welchem es sich um ein Protein oder ein anderes herzustellendes Polypeptid handelt, ist.

18. Polypeptid nach Anspruch 17, wobei *Polypeptid* ausgewählt ist aus der Gruppe, bestehend aus Aprotinin, Gewebefaktorweghemmer oder anderen Proteasehemmern, insulinartigem Wachstumsfaktor I oder II, Human- oder Rinderwachstumshormon, Interleukin, Gewebeplasminogenaktivator, Proglucagon und von Proglucagon abgeleiteten Polypeptiden, Glucagon, glucagonartigem Peptid-1, glucagonartigem Peptod-2, GRPP, Faktor VII, Faktor VIII, Faktor XIII, von Plättchen abgeleitetem Wachstumsfaktor, Enzymen, Insulin oder einem Insulinvorläufer.

19. Polypeptid nach Anspruch 18, wobei die von Proglucagon abgeleiteten Polypeptide ausgewählt sind aus der Gruppe, bestehend aus GLP-1(1-45), GLP-1(1-39), GLP-1(1-38), GLP-1(1-37), GLP-1(1-36), GLP-1(1-35), GLP-1(1-34), GLP-1(7-45), GLP-1(7-39), GLP-1(7-38), GLP-1(7-37), GLP-1(7-36), GLP-1(7-35) und GLP-1(7-34).

20. Polypeptid nach Anspruch 18, wobei das *Polypeptid* Insulin oder ein Insulinvorläufer, vorzugsweise ausgewählt aus der Gruppe, bestehend aus B(1-29)-A(1-21); B(1-27-Asp-Lys)-Ala-Ala-Lys-A(1-21); B(1-29)-Ala-Ala-Lys-A(1-21); und B(1-29)-Ser-Asp-Asp-Ala-Lys-A(1-21)
ist.

21. Rekombinanter Expressionsvektor, der in Hefe replizieren kann und ein DNA-Konstrukt nach einem der vorangehenden Ansprüche 1-16 trägt, das funktionsfähig an eine Promotersequenz, vorzugsweise die TPI-Promotersequenz, sowie an eine Terminatorsequenz, vorzugsweise die TPI-Terminatorsequenz gebunden ist, die zur Expression in Hefe geeignet sind.

22. Hefezelle, die ein heterologes Protein exprimieren kann und mit einem Vektor nach Anspruch 21 transformiert wird.

23. Hefezelle nach Anspruch 22, die zu einer beliebigen der Spezies *Saccharomyces cerevisiae, Saccharomyces kluyveri, Schizisaccharomyces pombe, Haresenula polymorpha* und *Yarrowia lipolytica* gehört.

24. Verfahren zur Herstellung eines heterologen Proteins, umfassend das Züchten einer Hefezelle nach einem der Ansprüche 22 oder 23 in einem geeigneten Medium unter Erhalt einer Expression und Sekretion des heterologen Proteins, wonach das Protein aus dem Kulturmedium isoliert wird.

25. Verfahren nach Anspruch 24, wobei die Aminosäuresequenz EEGEPK von dem heterologen Protein durch ein Verfahren unter Beteiligung der Behandlung mit einem proteolytischen Enzym, das für eine basische Aminosäure spezifisch ist, entfernt wird.

26. Verfahren nach Anspruch 25, wobei das proteolytische Enzym ausgewählt ist aus der Gruppe, bestehend aus Trypsin und Protease I von *Achromocacter lyticus.*

## Revendications

1. Produit de synthèse d'ADN codant pour une séquence polypeptidique ayant la structure suivante SP-LP-EEGEPK-*polypeptide*
où SP est une séquence d'ADN codant pour un peptide signal,
LP est une séquence d'ADN codant pour un peptide leader, et
EEGEPK est une séquence d'acides aminés positionnée comme une extension N-terminale du *polypeptide* qui suit qui est une protéine ou autre polypeptide à produire.

2. Produit de synthèse d' ADN selon la revendication précédente, dans lequel SP est une séquence d'ADN sélectionnée parmi le groupe de séquences d'ADN codant pour le peptide signal du facteur α de S. cerevisiae, le peptide signal de l'amylase salivaire de souris, le peptide signal d'une carboxypeptidase de levure, le peptide signal de la protéase aspartique 3 de levure (YAP3), ou le peptide signal de BAR1 de levure.

3. Produit de synthèse d'ADN selon l'une quelconque des revendications précédentes, dans lequel SP est une séquence d'ADN codant pour le peptide signal de la protéase aspartique 3 de levure (YAP3).

4. Produit de synthèse d'ADN selon l'une quelconque des revendications précédentes, dans lequel LP est une séquence d'ADN codant pour un peptide leader synthétique de 43 acides aminés ou plus.

5. Produit de synthèse d'ADN selon l'une quelconque des revendications précédentes, dans lequel LP est un séquence d'ADN codant pour un peptide leader synthétique ayant la formule X1-PIDDTESNTTSVNLMADDTES-X2-FATNTT-X3-LDW-X4-LISMAKR
où X1 est au moins un acide aminé sélectionné parmi le groupe constitué de A, D, E, F, G, H, I, K, L, M, N, P, Q, R, S, T, V, et Y,
X2 est une liaison peptidique ou un ou plusieurs acides aminés sélectionnés parmi le groupe constitué de A, D, E, F, G, H, I, K, L, M, N, P, Q, R, S, T, V, Y,
X3 est compris entre 1 et 10 acides aminés sélectionnés parmi le groupe constitué de A, D, E, F, G, H, I, K, L, M, N, P, Q, R, S, T, V, Y, et
X4 est une liaison peptidique ou un ou deux acides aminés sélectionnés parmi le groupe constitué de A, D, E, F, G, H, I, K, L, M, N, P, Q, R, S, T, V, Y.

6. Produit de synthèse d'ADN selon la revendication 5, dans lequel X1 est un acide aminé sélectionné parmi le groupe constitué de Q et S.

7. Produit de synthèse d'ADN selon la revendication 5, dans lequel X2 est un acide aminé sélectionné parmi le groupe constitué de R et A.

8. Produit de synthèse d'ADN selon la revendication 5, dans lequel X3 est une séquence de deux à cinq acides aminés sélectionnés parmi le groupe constitué de LA, LAGG, SVGG, NSGG,LADGG, LADD, et LAGD.

9. Produit de synthèse d'ADN selon la revendication 5, dans lequel X4 est un acide aminé sélectionné parmi le groupe constitué de N et G.

10. Produit de synthèse d'ADN selon l'une quelconque des revendications 1 à 5, dans lequel LP est une séquence d'ADN codant pour un peptide leader synthétique sélectionné parmi le groupe constitué de

11. Produit de synthèse d'ADN selon l'une quelconque des revendications 1, 2, 3 ou 4, dans lequel LP est une séquence d'ADN codant pour le peptide leader synthétique (LA19) :

12. Produit de synthèse d'ADN selon l'une quelconque des revendications précédentes, dans lequel le *polypeptide* est une protéine hétérologue.

13. Produit de synthèse d'ADN selon la revendication 12, dans lequel la protéine hétérologue est sélectionnée parmi le groupe constitué d'aprotinine, inhibiteur de la voie du facteur tissulaire ou autres inhibiteurs de protéase, facteur de croissance I ou II analogue à l'insuline, hormone de croissance humaine ou bovine, interleukine, activateur tissulaire du plasminogène, proglucagon et polypeptides dérivés de proglucagon, glucagon, peptide-1 analogue au glucagon, peptide 2 analogue au glucagon, GRPP, Facteur VII, Facteur VIII, Facteur XIII, facteur de croissance dérivé de plaquettes, enzymes, insuline ou un précurseur d'insuline.

14. Produit de synthèse d'ADN selon la revendication précédente, dans lequel les polypeptides dérivés de proglucagon sont sélectionnés parmi le groupe constitué de GLP-1(1-45), GLP1(1-39), GLP-1(1-38), GLP-1(1-37), GLP-1(1-36), GLP-1(1-35), GLP-1(1-34), GLP1(7-45), GLP-1(7-39), GLP-1(7-38), GLP-1(7-37), GLP-1(7-36), GLP-1(7-35), et GLP-1(7-34).

15. Produit de synthèse d'ADN selon la revendication 13, dans lequel la protéine hétérologue est une insuline ou un précurseur d'insuline, de préférence sélectionnée parmi le groupe constitué de B(1-29)-A(1-21), B(1-27-Asp-Lys)-Ala-Ala-Lys-A(1-21), B(1-29)Ala-Ala-Lys-A(1-21), et B(1-29)-Ser-Asp-Asp-Ala-Lys-A(1-21).

16. Produit de synthèse d'ADN décrit sur la figure 2 annexée.

17. Polypeptide ayant la séquence EE-GEPK-*polypeptide*, dans lequel EEGEPK est l'extension N-terminale du *polypeptide* qui suit qui est une protéine ou autre polypeptide à produire.

18. Polypeptide selon la revendication 17, dans lequel le *polypeptide* est sélectionné parmi le groupe constitué d'aprotinine, inhibiteur de la voie du facteur tissulaire ou autres inhibiteurs de protéase, facteur de croissance l ou II analogue à l'insuline, hormone de croissance humaine ou bovine, interleukine, activateur tissulaire du plasminogène, proglucagon et des polypeptides dérivés de proglucagon, glucagon, peptide-1 analogue au glucagon, peptide 2 analogue au glucagon, GRPP, Facteur VII, Facteur VIII, Facteur XIII, facteur de croissance dérivé de plaquettes, enzymes, insuline ou un précurseur d'insuline.

19. Polypeptide selon la revendication 18, dans lequel les polypeptides dérivés de progaglucon sont sélectionnés parmi le groupe constitué de GLP-1(1-45), GLP1(1-39), GLP-1(1-38), GLP-1(1-37), GLP-1(1-36), GLP-1(1-35), GLP-1(1-34), GLP1(7-45), GLP-1(7-39), GLP-1(7-38), GLP-1(7-37), GLF-1(7-36), GLP-1(7-35), et GLP-1(7-34),

20. Polypeptide selon la revendication 18, dans lequel le *polypeptide* est une insuline ou un précurseur d'insuline, sélectionnée parmi le groupe constitué de B(1-29)-A(1-21), B(1-27-Asp-Lys)-Ala-Ala-Lys-A(1-21), B(1-29)Ala-Ala-Lys-A(1-21), et B(1-29)-Ser-Asp-Asp-Ala-Lys-A(1-21).

21. Vecteur d'expression recombinant qui est capable de se répliquer dans une levure et qui transporte un produit de synthèse d'ADN selon l'une quelconque des revendications 1 à 16, relié de manière opérationnelle à une séquence promoteur, de préférence la séquence promoteur de TPI, ainsi qu'une séquence de terminaison, de préférence la séquence de terminaison de TPI, qui sont adaptées à une expression dans une levure.

22. Cellule de levure qui est capable d'exprimer une protéine hétérologue et qui est transformée par un vecteur selon la revendication 21.

23. Cellule de levure selon la revendication 22, et qui appartient à l'une quelconque des espèces Saccharomyces cerevisiae, Saccharomyces kluyveri, Schizosaccharomyces pombe, Hansenula polymorpha, et Yarrowia lipolytica.

24. Procédé pour produire une protéine hétérologue, comportant 1a culture d'une cellule de levure selon l'une quelconque des revendications 22 ou 23 dans un milieu adapté pour obtenir l'expression et la sécrétion de la protéine hétérologue, après quoi la protéine est isolée du milieu de culture.

25. Procédé selon la revendication 24, dans lequel la séquence d'acides aminés EEGEPK est retirée de la protéine hétérologue récupérée par un processus impliquant un traitement à l'aide d'une enzyme protéolytique qui est spécifique à un acide aminé basique.

26. Procédé selon la revendication 25, dans lequel l'enzyme protéolytique est sélectionnée parmi le groupe constitué de trypsine et de la protéase I de Achromobacter lyticus.
